# EUROPÄISCHE PATENTSCHRIFT

(11) **EP 4 636 270 B1**
(45) Veröffentlichungstag und Bekanntmachung des Hinweises auf die Patenterteilung: **18.11.2026**
(21) Anmeldenummer: 25170087.8
(22) Anmeldetag: 11.04.2025
(51) Int. Cl.: F16C 7/02, A61B 18/14, A61B 17/29, A61B 17/00, A61B 18/00

(54) **KRAFTÜBERTRAGUNGSELEMENT, CHIRURGISCHES INSTRUMENT UND VERFAHREN ZUR HERSTELLUNG DES KRAFTÜBERTRAGUNGSELEMENTS**
FORCE TRANSMISSION MEMBER, SURGICAL INSTRUMENT, AND METHOD FOR MANUFACTURING FORCE TRANSMISSION MEMBER
ÉLÉMENT DE TRANSMISSION DE FORCE, INSTRUMENT CHIRURGICAL ET PROCÉDÉ DE FABRICATION D'ÉLÉMENT DE TRANSMISSION DE FORCE

(30) Priorität: 18.04.2024 DE 102024110881
(43) Veröffentlichungstag der Anmeldung: 22.10.2025
(73) Patentinhaber: Karl Storz SE & Co. KG, 78532 Tuttlingen (DE)
(72) Erfinder: MERZ, Robin, 78532 Tuttlingen (DE); HAHN, Benedikt, 78532 Tuttlingen (DE); MOOSMANN, Nico, 78532 Tuttlingen (DE); KÄRCHER, Daniel, 78532 Tuttlingen (DE)

(56) Entgegenhaltungen:
- WO-A1-2022/268846
- US-A1- 2017 333 115
- US-A1- 2022 160 420

## Beschreibung

### GEBIET DER ERFINDUNG

Die vorliegende Erfindung betrifft ein Kraftübertragungselement für chirurgische Instrumente, insbesondere eine Zugstange für ein endoskopisches Instrument. Ferner betrifft die vorliegende Erfindung ein chirurgisches Instrument umfassend ein derartiges Kraftübertragungselement und ein Verfahren zur Herstellung eines derartigen Kraftübertragungselements.

### TECHNISCHER HINTERGRUND

Chirurgische Instrumente weisen oftmals eine mechanische Lagerung, insbesondere eine Verdrehsicherung oder eine Axialführung mit Anschlag, auf. Zudem weisen chirurgische Instrumente oftmals eine elektrische Trennung (Isolierung) auf. Die mechanische Lagerung und elektrische Trennung werden durch separate Bauteile oder Elemente des chirurgischen Instruments realisiert.

Beispielsweise sind chirurgische Rohrschaftinstrumente (z. B. Endoskope) als wiederverwendbare Rohrschaftinstrumente ausgebildet, so lassen sich je nach Konstruktionen des Instruments insbesondere der Schaft, der Isolationsschaft und das Kraftübertragungselement zum Reinigen des Instruments vollständig voneinander trennen. Nach der Reinigung wird das Instrument wieder montiert und sterilisiert. Die Erfahrung zeigt, dass Schleifkontakte, wie sie an Polen vorgesehen sind, durch die
Reinigung an Leistungsfähigkeit verlieren, zum Beispiel durch Oxidation. Daher sind Schleifkontakte zu minimieren.

Weiter beispielsweise weisen bipolare betriebene chirurgische Rohrschaftinstrumente (z. B. Endoskope mit bipolarem Werkzeug / Zubehör) zwei elektrische Leitungen auf, die üblicherweise durch zwei gegeneinander elektrisch isolierte Bauteile oder Elemente des chirurgischen Rohrschaftinstruments gebildet sind. Bei bipolar betriebenen chirurgischen Rohrschaftinstrumenten sind an dem Zubehör des chirurgischen Rohrschaftinstruments zwei Elektroden (Aktivelektrode und Neutralelektrode) angeordnet. Dabei kann von einer ersten Elektrode (Aktivelektrode) aus hochfrequentem Wechselstrom direkt gegenüber der zweiten Elektrode in das Zielgewebe eingebracht werden. WO 2022/268846 A1 offenbart ein Kraftübertragungselement mit kombinierter mechanischer Lagerung und elektrischer Trennung, sowie ein chirurgisches Instrument umfassend das Kraftübertragungselement und ein Verfahren zur Herstellung des Kraftübertragungselements.

### ZUSAMMENFASSUNG DER ERFINDUNG

Vor diesem Hintergrund liegt der vorliegenden Erfindung die Aufgabe zugrunde, ein verbessertes medizinisches Instrument mit mechanischer Lagerung und elektrischer Trennung bereitzustellen.

Erfindungsgemäß wird diese Aufgabe durch ein Kraftübertragungselement mit den Merkmalen des Patentanspruchs 1 und/oder durch ein chirurgisches Instrument mit den Merkmalen des Patentanspruchs 9 und/oder durch ein Verfahren mit den Merkmalen des Patentanspruchs 11 gelöst.

Dementsprechend ist gemäß einem ersten Aspekt der vorliegenden Erfindung ein Kraftübertragungselement für chirurgische Instrumente, insbesondere eine Zugstange für ein endoskopisches Instrument, vorgesehen. Das Kraftübertragungselement umfasst einen Stab und eine Elektrode. Der Stab ist potentialfrei, zur Kraftübertragung ausgebildet und weist zumindest eine lokale Vertiefung auf. Die Elektrode erstreckt sich über eine äußere Oberfläche der Vertiefung und zumindest abschnittsweise in axialer Richtung entlang der Vertiefung, wobei die Elektrode in Kontakt mit einem elektrischen Leiter ist, der sich innerhalb des Stabs erstreckt. Ein Ende, insbesondere ein proximales Ende, des Stabs weist ein elektrisch isoliertes Formschlusselement auf, das zum Eingriff mit einem Lagerelement bzw. Bedienelement eines chirurgischen Instruments ausgebildet ist.

Ferner ist gemäß einem zweiten Aspekt der vorliegenden Erfindung ein chirurgisches Instrument, insbesondere ein chirurgisches Rohrschaftinstrument mit einem Kraftübertragungselement gemäß dem ersten Aspekt der vorliegenden Erfindung, einem Rohrschaft, einer Betätigungsschnittstelle, einem Lagerelement und einem Werkzeug vorgesehen. In dem Rohrschaft ist das Kraftübertragungselement aufgenommen. Die Betätigungsschnittstelle ist zum Betätigen des Kraftübertragungselements ausgebildet. Das Lagerelement ist in der Betätigungsschnittstelle integriert und koppelt das Formschlusselement des Kraftübertragungselements. Das Werkzeug ist mittels des Kraftübertragungselements bewegbar.

Außerdem ist gemäß einem dritten Aspekt der vorliegenden Erfindung ein Verfahren zur Herstellung eines Kraftübertragungselements für chirurgische Instrumente, insbesondere eines Kraftübertragungselements gemäß dem ersten Aspekt der vorliegenden Erfindung, vorgesehen, das die folgenden Schritte umfasst:
Bereitstellen eines potentialfreien, zur Kraftübertragung ausgebildeten Stabs, der zumindest eine lokale Vertiefung und ein Ende, insbesondere ein proximales Ende, mit einem elektrisch isolierten Formschlusselement aufweist, das zum Eingriff mit einem Lagerelement eines chirurgischen Instruments ausgebildet ist.
Aufbringen einer Elektrode, die sich über eine äußere Oberfläche der Vertiefung und zumindest abschnittsweise in axialer Richtung entlang der Vertiefung erstreckt, wobei die Elektrode in Kontakt mit einem elektrischen Leiter gebracht wird, der sich innerhalb des Stabs erstreckt.

Die der vorliegenden Erfindung zugrunde liegende Idee besteht darin, Strom und eine Zug-/Druckkraft über das Kraftübertragungselement bzw. Zugstange in die Betätigungsschnittstelle zu transportieren, wobei der Stab selbst sowie das proximale Ende keinen Strompol führen. Der Stab selbst steht nicht unter elektrischer Spannung.

Der Stab kann zumindest teilweise aus einem metallischen Werkstoff wie beispielsweise Stahl oder Edelstahl gefertigt sein. Ferner ist wenigstens ein potentialfreier, zum Beispiel elektrisch isolierender, Bereich oder Abschnitt vorgesehen. Der Stab kann insbesondere ausgebildet sein, eine Kraft in axialer Richtung (Zug oder Druck) und zusätzlich oder alternativ ein Drehmoment zu Übertragen. Die Kraft in axialer Richtung bzw. das Drehmoment kann von der Betätigungsschnittstelle über das Lagerelement auf den Stab übertragen werden und durch den Stab an ein Zubehör oder Werkzeug des chirurgischen Instruments weitergeleitet werden, um das Zubehör bzw. Werkzeug zu bewegen.

Die Vertiefung in dem Stab dient als Anbringungsbereich für die Elektrode. Die Vertiefung kann in einem urformenden Verfahren vorgesehen werden oder durch ein spanendes Verfahren wie beispielsweise Fräsen in den Stab eingebracht werden.

Die Funktion der Stromübertragung wird dabei durch den elektrischen Leiter und die Elektrode bereitgestellt. Der elektrische Leiter erstreckt sich in axialer Richtung in dem Stab und ist elektrisch durch den Stab von einer Umgebung getrennt. Optional kann der elektrische Leiter eine elektrisch isolierende Ummantelung bzw. Beschichtung aufweisen. Zum Beispiel kann der elektrische Leiter als Stromkabel ausgebildet sein. Für bestimmte Anwendungen kann beispielsweise eine zweite Elektrode vorgesehen sein. Die zweite Elektrode kann sich über eine äußere Oberfläche einer zweiten Vertiefung und zumindest abschnittsweise in axialer Richtung entlang der zweiten Vertiefung erstrecken. Insbesondere in einem bipolaren Betrieb des chirurgischen Werkzeugs kann der elektrische Strom über das Kraftübertragungselement geleitet werden, indem einer der beiden Pole zur Leitung des elektrischen Stroms mit der Elektrode und der andere der beiden Pole zur Leitung des elektrischen Stroms mit der zweiten Elektrode elektrisch verbunden wird. Beide Elektroden sind jeweils mit einem eigenen elektrischen Leiter verbunden, wobei die beiden elektrischen Leiter elektrisch getrennt voneinander in dem potentialfreien Stab angeordnet sind. Das heißt, der Stab isoliert die beiden Elektroden voneinander. Insbesondere isoliert der Stab die Elektrode bzw. die beiden Elektroden von dem Formschlusselement. Folglich steht das Ende bzw. das Formschlusselement nicht in elektrischem Kontakt zu dem elektrischen Leiter oder der Elektrode.

Das Kraftübertragungselement ist in den Rohrschaft des chirurgischen Instruments eingeschoben. Dabei ist das Kraftübertragungselement zumindest an dem Ende, insbesondere dem proximalen Ende, des Stabs in dem chirurgischen Instrument gelagert bzw. geführt. Insbesondere ist der Stab in dem Rohrschaft derart gelagert bzw. geführt, dass das Kraftübertragungselement sich gegenüber dem Rohrschaft axial verschieben oder rotieren kann. Somit kann das Kraftübertragungselement eine axiale Kraft (Zug oder Druck) und zusätzlich oder alternativ ein Drehmoment innerhalb des Rohrschafts und relativ zu diesem übertragen.

Die Betätigungsschnittstelle dient zum Betätigen des Kraftübertragungselements, indem eine axiale Kraft oder ein Drehmoment auf das Kraftübertragungselement aufgebracht wird. Das Lagerelement der Betätigungsschnittstelle greift das Formschlusselement des Kraftübertragungselements in einem betriebsbereiten Zustand. Wird die Betätigungsschnittstelle betätigt, überträgt das Lagerelement die axiale Kraft oder das Drehmoment auf das Formschlusselement des Stabs und betätigt folglich das Kraftübertragungselement.

Das Werkzeug, das insbesondere in Form eines austauschbaren Zubehörs ausgebildet sein kann, wird durch die über das Kraftübertragungselement innerhalb und gegenüber des Rohrschafts übertragene axiale Kraft bzw. Drehmoment bewegt. Das Werkzeug kann als Klemme, Zange, Pinzette, Greifer, Schere und dergleichen ausgebildet sein. Insbesondere kann das Werkzeug zudem ausgebildet sein, monopolar oder bipolar betrieben zu werden, um beispielsweise Gewebe zu schneiden, zu veröden und dergleichen.

Sofern das chirurgische Instrument durch einen Nutzer wie beispielsweise einen Chirurgen geführt werden soll, umfasst das chirurgische Instrument einen Handgriff als Handhabe für den Nutzer. Die Betätigungsschnittstelle überträgt sodann eine Kraft bzw. ein Drehmoment, die/das von dem Nutzer am Handgriff über eine geeignete Betätigung (z. B. beweglicher Griffschenkel) aufgebracht wird, and das Kraftübertragungselement.

Sofern das chirurgische Instrument an einen Roboter anschließbar ist, umfasst das chirurgische Instrument eine entsprechende Verbindungsschnittstelle für den Roboter, der eine axiale Kraft bzw. ein Drehmoment über die Betätigungsschnittstelle auf das Kraftübertragungselement aufbringen kann.

Das erfindungsgemäße Kraftübertragungselement mit Stab und Elektrode inkl. elektrischem Leiter stellt eine funktionale Trennung von mechanischer Kraftübertragung und Stromübertragung bereit, da jede Komponente lediglich eine der beiden Funktionen erfüllt. Zudem kann durch das erfindungsgemäße Kraftübertragungselement ein vorbestimmter Bauraum und die erforderlichen Kriechstrecken zwischen der Elektrode und den nichtstromführenden Komponenten bzw. Bauteilen wie beispielsweise dem Formschlusselement des Stabs eingehalten werden. Ferner kann ein Montageaufwand erheblich reduziert werden.

Vorteilhafte Weiterbildungen und Ausgestaltungen der vorliegenden Erfindung sind Gegenstand der entsprechenden abhängigen Patentansprüche.

Gemäß einer Weiterbildung der vorliegenden Erfindung weist der Stab einen Mittelabschnitt und einen zwischen dem Mittelabschnitt und dem Ende angeordneten Kontaktabschnitt auf, in welchem die zumindest eine Vertiefung angeordnet ist, wobei der Kontaktabschnitt eine Hülse aus keramischem Werkstoff aufweist. Die Hülse kann dabei als Grundgerüst für die anderen Komponenten dienen. Die Hülse ist insbesondere derart mechanisch stabil konstruiert, dass die Hülse ohne eine Verwendung von sonstigen elektrisch isolierenden Werkstoffen, zum Beispiel Kunststoff, als tragendes Bauteil verwendbar ist. Die Hülse kann beispielsweise gemäß einer Ausführungsform als, insbesondere im Wesentlichen, zylinderförmiger Körper ausgebildet sein, in welchem zwei oder mehr Bohrungen enthalten sind. Die Grundfläche der Zylinderform kann kreisförmig sein, es sind jedoch auch andere Grundformen denkbar. Es kann sich um einen massiven Körper oder um einen Hohlkörper handeln. Zusätzliche Ausnehmungen, Formänderungen im Verlauf, Hinterschnitte oder dergleichen können darin vorgesehen sein. Darüber hinaus kann der Stab einen flexiblen, zum Beispiel elastisch biegsamen oder artikulierbaren, Abschnitt aufweisen. Weiterhin kann die Hülse aus einem Glaswerkstoff hergestellt sein.

Die Vertiefung des Stabs kann beispielsweise umfänglich ausgebildet sein.

Optional kann die Elektrode in einem 2-Komponenten-Verfahren direkt generativ in die Hülse bzw. in den Kontaktabschnitt integriert werden. Dadurch können sich ein Montageaufwand weiter verringern, Spalte reduzieren und/oder Toleranzprobleme reduzieren. Darüber hinaus können sich eine Anzahl an elektrischen Übergängen, etwa Lötpunkte, und/oder Verbindungsstellen reduzieren. Ferner kann die Hülse einen Hinterschnitt aufweisen, ohne eine Nachbearbeitung der Hülse ausführen zu müssen.

Ein keramischer Werkstoff bietet eine gute mechanische Stabilität und exzellente elektrische Isolationseigenschaften.

Gemäß einer weiteren Weiterbildung weist der Kontaktabschnitt eine Drahteinrichtung zur Kraftübertragung zwischen dem Mittelabschnitt und dem Ende auf, wobei sich die Drahteinrichtung in axialer Richtung entlang des Stabs erstreckt und zumindest abschnittsweise von der Hülse umgeben ist. Beispielsweise ist die Drahteinrichtung radial von der Hülse umschlossen. Die Drahteinrichtung ist durch den keramischen Werkstoff der Hülse elektrisch von der Elektrode und dem elektrischen Leiter isoliert. Somit kann die Drahteinrichtung zum Beispiel aus einem Metall oder vergleichbaren Werkstoffen hergestellt sein, ohne dabei auf elektrisch nicht leitfähige Werkstoffe beschränkt zu sein.

Die Drahteinrichtung umfasst vorzugsweise zwei Zugdrähte, um insbesondere eine Zugkraft und gegebenenfalls ein Biegemoment zwischen dem Ende des Stabs und dem Mittelabschnitt zu übertragen. Dabei können sich die zwei Zugdrähte jeweils in separaten Bohrungen durch die Hülse erstrecken.

Gemäß einer weiteren Weiterbildung ist die Drahteinrichtung mit dem Formschlusselement und dem Mittelabschnitt des Stabs jeweils stoffschlüssig verbunden, wobei die Hülse durch die Drahteinrichtung zwischen dem Formschlusselement und dem Mittelabschnitt zumindest formschlüssig befestigt ist. Darüber hinaus kann die Hülse an einer Kontaktstelle zu dem Ende bzw. dem Mittelabschnitt geklebt sein. Zum Beispiel kann eine Stirnfläche der Hülse mit einer Stirnfläche des Endes verklebt sein. Alternativ oder zusätzlich kann die Hülse in den Mittelabschnitt des Stabs eingeklebt sein.

Gemäß einer weiteren Weiterbildung ist die Drahteinrichtung mit dem Formschlusselement und dem Mittelabschnitt jeweils stoffschlüssig, insbesondere mittels einer Schweißverbindung oder mittels eines kapillar wirkenden Klebstoffs, verbunden.

Gemäß einer weiteren Weiterbildung ist die Hülse durch ein generatives Fertigungsverfahren oder durch Spritzgießen einteilig hergestellt. Auf diese Weise kann ein nachträgliches Bearbeiten der Hülse, insbesondere ein spanendes Verfahren, zur Schaffung der Vertiefung entfallen. Mithilfe des generativen Fertigungsverfahrens bzw. der additiven Fertigung können komplexe Innenkonturen und ein hohes Aspektverhältnis aus Bohrungstiefe im Verhältnis zu Bohrungsdurchmesser bzw. gesamte Breite im Verhältnis zur gesamten Länge bewirkt werden. Damit kann eine schlankere Bauform realisiert werden, was insbesondere einen Durchmesser der Hülse sowie des Stabs von weniger als etwa 2,5 mm ermöglicht.

Gemäß einer weiteren Weiterbildung weist die Vertiefung wenigstens einen im Wesentlichen ebenen Abschnitt parallel zu der axialen Richtung des Stabs auf, welcher gegenüber einer äußeren Oberfläche des Stabs in radialer Richtung weiter innen liegt und mit der Elektrode aneinander anliegend, insbesondere flächig anliegend, kontaktiert ist.

Gemäß einer weiteren Weiterbildung ist das Formschlusselement kugelförmig ausgebildet. Ein Durchmesser des kugelförmigen Formschlusselements beträgt höchstens einen Durchmesser des Stabs. Zum Beispiel ragt das Formschlusselement in axialer Richtung von dem Stab ab. Das Formschlusselement ist beispielsweise aus einem Metall hergestellt.

Gemäß einer weiteren Weiterbildung ist der Rohrschaft als Rundrohr ausgebildet. Der Stab des Kraftübertragungselements ist als Rundstab ausgebildet. Die Hülse erstreckt sich zumindest abschnittsweise zirkulär entlang der Oberfläche des Stabs. Das Kraftübertragungselement ist konzentrisch in dem Rohrschaft angeordnet.

Der als Rundstab ausgebildete Stab hat einen im Wesentlichen kreisrunden Querschnitt mit im Wesentlichen konstanten Durchmesser entlang der axialen Richtung, außer im Bereich der Vertiefung.

Der als Rundrohr ausgebildete Rohrschaft hat einen im Wesentlichen kreisrunden Querschnitt mit im Wesentlichen konstanten Durchmesser entlang der axialen Richtung. Das Kraftübertragungselement mit als Rundstab ausgebildetem Stab ist in dem als Rundrohr ausgebildeten Rohrschaft konzentrisch angeordnet. Dabei greift das Lagerelement das Formschlusselement formschlüssig, um das Kraftübertragungselement mit dem als Rundstab ausgebildeten Stab in dem als Rundrohr ausgebildeten Rohrschaft zu sichern.

Das Lagerelement des chirurgischen Elements greift das Formschlusselement des Stabs derart, dass eine axiale Verschiebung und ggf. eine Rotation des Stabs gegenüber dem Rohrschaft möglich ist.

Bei einer Ausführungsform kann eine Rotation des Stabs gegenüber dem Rohrschaft beispielsweise durch eine Längsnut oder einen abgeflachten Bereich des Stabs blockiert oder begrenzt sein. Somit kann eine ungewollte Rotation des gesamten Kraftübertragungselements auch gegenüber dem Werkzeug bzw. Zubehör des chirurgischen Elements vermieden und somit eine einwandfreie Funktion des Werkzeugs/Zubehörs sichergestellt werden.

Das derart ausgebildete Kraftübertragungselement und chirurgische Instrument sind vorteilhaft besonders einfach zu fertigen.

Gemäß einer Weiterbildung der vorliegenden Erfindung umfasst das Aufbringen der Elektrode ein Abisolieren und Anschweißen, Löten und/oder Krimpen des elektrischen Leiters an die Elektrode. Bei Bedarf kann der elektrische Leiter zudem auf eine geeignete Länge gekürzt werden. Insbesondere wird der abisolierte Abschnitt des elektrischen Leiters an eine Innenseite der Elektrode angeschweißt.

Gemäß einer Weiterbildung der vorliegenden Erfindung weist der Stab einen Mittelabschnitt und einen zwischen dem Mittelabschnitt und dem Ende angeordneten Kontaktabschnitt auf, in welchem die zumindest eine Vertiefung angeordnet ist, wobei eine Drahteinrichtung durch eine in dem Kontaktabschnitt vorgesehene Hülse aus keramischem Werkstoff hindurchgeschoben wird. Beispielsweise wird zunächst das Ende an dem Kontaktabschnitt montiert bevor der Mittelabschnitt an den Kontaktabschnitt montiert wird.

Gemäß einer Weiterbildung der vorliegenden Erfindung umfasst das Verfahren ferner den Schritt stoffschlüssiges Verbinden der Drahteinrichtung mit dem Formschlusselement.

Gemäß einer Weiterbildung der vorliegenden Erfindung wird nach dem stoffschlüssigen Verbinden die Drahteinrichtung mit der aufgeschobenen Hülse an den Mittelabschnitt stoffschlüssig verbunden, sodass der elektrische Leiter innerhalb der Hülse aufgenommen wird.

In manchen Ausgestaltungen der Erfindung kann der Schritt des Bereitstellens des Stabs einen Schritt des Schaffens der lokalen Vertiefung in dem Stab umfassen, insbesondere durch Umformen, beispielweise Pressen, oder durch Zerspanen, beispielsweise Fräsen oder Drehen.

Optional können das Ende und ggf. der Kontaktabschnitt nach dem stoffschlüssigen Verbinden mit einem kapillar wirkenden Klebstoff versehen werden. Zum Beispiel kann das Ende und ggf. der Kontaktabschnitt in einen kapillar wirkenden Klebstoff getaucht werden. Durch die Kapillarwirkung können Spalte mit Klebstoff gefüllt werden.

Die obigen Ausgestaltungen und Weiterbildungen lassen sich, sofern sinnvoll, beliebig miteinander kombinieren. Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmale der Erfindung. Insbesondere wird dabei der Fachmann auch Einzelaspekte als Verbesserungen oder Ergänzungen zu der jeweiligen Grundform der vorliegenden Erfindung hinzufügen.

### INHALTSANGABE DER ZEICHNUNG

Die vorliegende Erfindung wird nachfolgend anhand der in den schematischen Figuren der Zeichnung angegebenen Ausführungsbeispiele näher erläutert. Es zeigen dabei:
- Fig. 1: eine Explosionsdarstellung einer Ausführungsform eines Kraftübertragungselements;
- Fig. 2: eine schematische Seitenansicht des Kraftübertragungselements aus Fig. 1 in einem montierten Zustand;
- Fig. 3: eine isometrische Ansicht eines proximalen Endes des Kraftübertragungselements;
- Fig. 4: eine transparente isometrische Ansicht einer Hülse des Kraftübertragungselements;
- Fig. 5: eine Seitenansicht einer Ausführungsform eines handgeführten chirurgischen Instruments;
- Fig. 6: einen Längsschnitt durch das handgeführte chirurgische Instrument im Bereich eines Lagerelements einer Betätigungsschnittstelle; und
- Fig. 7: ein Ablaufdiagramm einer Ausführungsform des Verfahrens zur Herstellung eines Kraftübertragungselements.

Die beiliegenden Figuren der Zeichnung sollen ein weiteres Verständnis der Ausführungsformen der Erfindung vermitteln. Sie veranschaulichen Ausführungsformen und dienen im Zusammenhang mit der Beschreibung der Erklärung von Prinzipien und Konzepten der Erfindung. Andere Ausführungsformen und viele der genannten Vorteile ergeben sich im Hinblick auf die Zeichnungen. Die Elemente der Zeichnungen sind nicht notwendigerweise maßstabsgetreu zueinander gezeigt.

In den Figuren der Zeichnung sind gleiche, funktionsgleiche und gleich wirkende Elemente, Merkmale und Komponenten - sofern nichts Anderes ausgeführt ist - jeweils mit denselben Bezugszeichen versehen.

### BESCHREIBUNG VON AUSFÜHRUNGSBEISPIELEN

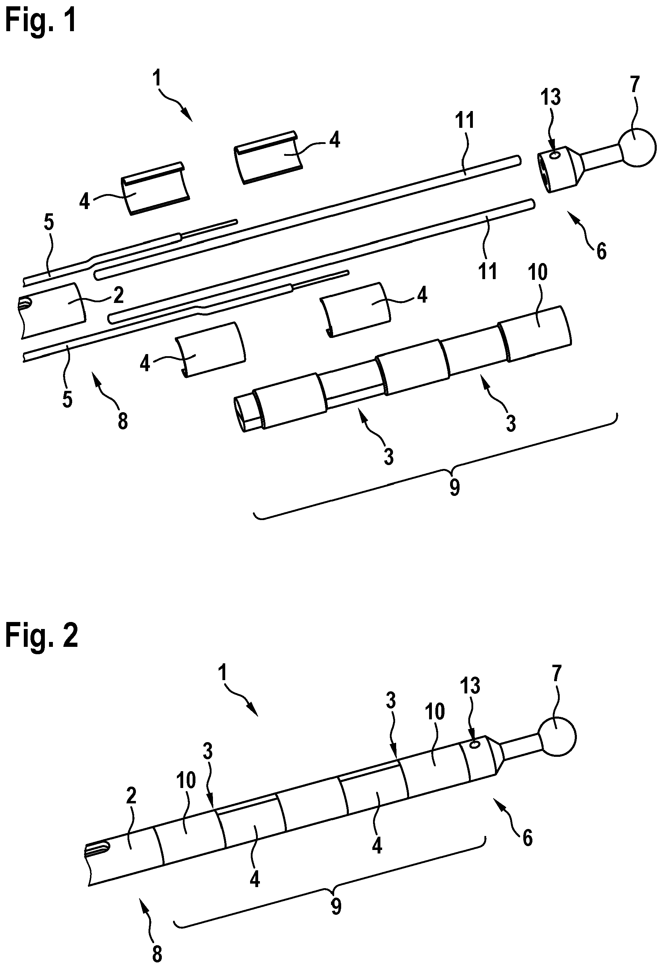

In den Figs. 1 und 2 ist eine Ausführungsform eines Kraftübertragungselements 1 illustriert. Im Speziellen zeigt Fig. 1 das Kraftübertragungselement 1 in einer Explosionsdarstellung.

Das beispielhafte Kraftübertragungselement 1 ist insbesondere als Zugstange für ein endoskopisches Instrument ausgebildet. Die Zugstange 1 umfasst einen Stab 2, zwei umfängliche Vertiefungen 3, zwei Elektroden 4, zwei elektrische Leiter 5, ein proximales Ende 6 mit einem Formschlusselement 7, eine Hülse 10 aus keramischem Werkstoff sowie eine Drahteinrichtung 11.

Der Stab 2 ist potentialfrei, zur Kraftübertragung ausgebildet und weist in einem Kontaktabschnitt 9 die zwei Vertiefungen 3 auf. Der Kontaktabschnitt 9 weist die Hülse 10 aus keramischem Werkstoff auf. Insbesondere weist die Hülse 10 die zwei umfänglichen Vertiefungen 3 auf. Weiterhin umfasst der Stab 2 einen Mittelabschnitt 8, wobei der Kontaktabschnitt 9 zwischen dem Mittelabschnitt 8 und dem proximalen Ende 6 angeordnet ist. Der Mittelabschnitt 8 kann dabei durch die Hülse 10 von den zwei Elektroden 4 isoliert sein.

Beispielhaft weisen die zwei Vertiefungen 3 einen im Wesentlichen ebenen Abschnitt parallel zu der axialen Richtung des Stabs 2 auf, welcher gegenüber einer äußeren Oberfläche des Stabs in radialer Richtung weiter innen liegt und mit der jeweiligen Elektrode 4 aneinander flächig anliegend kontaktiert ist.

Die Drahteinrichtung 11 enthält beispielsweise zwei Zugdrähte. Diese Zugdrähte 11 erstrecken sich jeweils in axialer Richtung entlang des Stabs 2 durch den Kontaktabschnitt 9 und ist zur Kraftübertragung zwischen dem Mittelabschnitt 8 und dem proximalen Ende 6 ausgebildet. Dabei sind die zwei Zugdrähte 11 von der Hülse 10 umgeben. Beide Zugdrähte 11 sind mit dem Formschlusselement 7 und dem Mittelabschnitt 8 des Stabs 2 jeweils mittels einer Schweißverbindung verbunden. Die Hülse 10 ist durch die Drahteinrichtung 11 zwischen dem Formschlusselement 7 und dem Mittelabschnitt 8 formschlüssig befestigt. Das proximale Ende 6 sowie der Mittelabschnitt 8 weisen jeweils zwei Aufnahmebereiche zur Aufnahme von Enden der zwei Zugdrähte 11 auf. Beispielsweise sind die zwei Aufnahmebereiche als Sackloch ausgebildet. Ferner enthält das proximale Ende 6 eine Aussparung 13, die sich in radialer Richtung erstreckt und zumindest eines der Sacklöcher kreuzt.

Die Hülse 10 enthält hier vier Bohrungen, die sich im Wesentlichen in axialer Richtung erstrecken, wobei zwei der vier Bohrungen zur Aufnahme der zwei elektrischen Leiter 5 und die anderen zwei der vier Bohrungen zur Aufnahme der zwei Zugdrähte 11 ausgebildet sind. Im Bereich der Vertiefung 3 enthält die Hülse 10 eine Aussparung, welche die Bohrung für den elektrischen Leiter 5 mit einer äußeren Oberfläche der Vertiefung 3 verbindet, damit der elektrische Leiter 5 durch die Aussparung aus der Hülse 10 herausragen kann. Die Hülse 10 ist durch ein generatives Fertigungsverfahren einteilig hergestellt.

Die zwei elektrischen Leiter 5 können zum Beispiel jeweils eine elektrisch isolierende Ummantelung bzw. Beschichtung aufweisen.

In der Fig. 2 ist eine Seitenansicht des Kraftübertragungselements in einem montierten Zustand schematisch dargestellt.

Die zwei Elektroden 4 erstrecken sich über die äußere Oberfläche der Vertiefung 3 und zumindest abschnittsweise in axialer Richtung entlang der Vertiefung 3. Dabei sind die Elektroden 4 jeweils in Kontakt mit einem der zwei elektrischen Leiter 5, der sich innerhalb des Stabs 2, insbesondere in der dafür vorgesehenen Bohrung der Hülse 10, erstreckt. Die zwei Elektroden 4 weisen jeweils zwei Halbschalen auf, die zusammen den Stab 2 umfänglich in der Vertiefung 3 umgeben. Das heißt, die Elektrode 4 ist beispielhaft in zwei Halbschalen unterteilt, die zusammen einen Ring bilden. Die Elektrode 4 liegt zirkulär und konzentrisch an dem Stab 2 im Bereich der Vertiefung 3 an.

Das Formschlusselement 7 des proximalen Endes 6 ist elektrisch isoliert. Beispielhaft ist das Formschlusselement 7 kugelförmig ausgebildet und aus einem Metall hergestellt. Das kugelförmige Formschlusselement 7 dient zum Eingriff mit einem Lagerelement 103 bzw. Bedienelement eines chirurgischen Instruments 100. Dabei ragt das Formschlusselement 7 in axialer Richtung von dem Stab 2 ab. Ein Durchmesser des Formschlusselements 7 ist geringer als ein Durchmesser des Stabs 2.

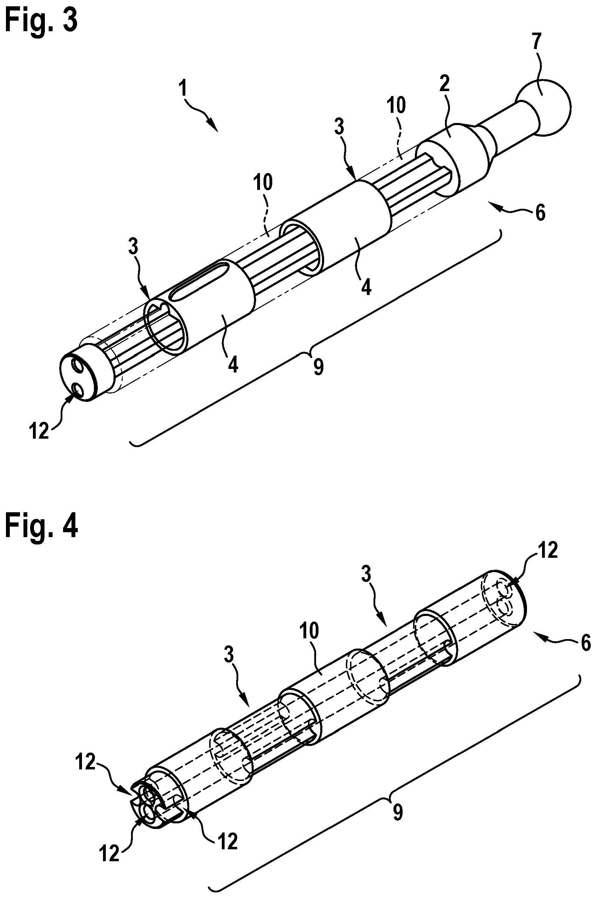

Fig. 3 zeigt eine isometrische Ansicht eines proximalen Endes 6 des Kraftübertragungselements 1. Das hier dargestellte Kraftübertragungselement 1 umfasst im Wesentlichen dieselben Merkmale wie die Ausführungsform nach den Figs. 1 und 2, sofern nichts Abweichendes beschrieben ist.

Die Hülse 10 ist transparent illustriert, um das Innere des Kontaktabschnitts 9 in einem montierten Zustand abzubilden.

Die Vertiefung 3 in dem Stab 2 ist zirkulär umlaufend und weist einen distalen Anschlag der Vertiefung 3 und einen proximalen Anschlag der Vertiefung 3 auf. Die zwei Elektroden 4 liegen zirkulär und konzentrisch an dem Stab 2 im Bereich der jeweiligen Vertiefung 3 an.

An einer Stirnseite des Kontaktabschnitts 9, insbesondere an dessen distaler Stirnseite, sind die zwei Bohrungen 12 für die zwei elektrischen Leiter 5 zu erkennen.

Fig. 4 zeigt eine transparente isometrische Ansicht einer Hülse 10 des Kraftübertragungselements 1. Insbesondere ist der komplexe innere Aufbau der Hülse 10 mit vier innenliegenden Bohrungen 12 zur Führung der zwei Zugdrähte und der zwei elektrischen Leiter 5 bzw. Kabel gezeigt.

Die Hülse 10 ist zum Beispiel einteilig aus einem keramischen Werkstoff durch ein generatives Fertigungsverfahren hergestellt. Bei der einteiligen Herstellung sind bereits zwei umfängliche Vertiefungen 3 berücksichtigt, die in axialer Richtung voneinander beabstandet angeordnet sind.

Zwei der vier innenliegenden Bohrungen 12 sind als Durchgangsbohrung für die zwei Zugdrähte in der axialen Richtung ausgebildet. Die anderen zwei der vier innenliegenden Bohrungen 12 sind für die zwei elektrischen Leiter vorgesehen und erstrecken sich ebenfalls in der axialen Richtung, wobei diese Bohrungen jeweils im Bereich der Vertiefung 3 enden/beginnen. Beispielsweise erstrecken sich die zwei Bohrungen 12 für die elektrischen Leiter ausgehend von der Vertiefung 3 nicht in Richtung eines proximalen Endes 6, sondern in Richtung eines distalen Endes des Kraftübertragungselements bzw. der Zugstange 1.

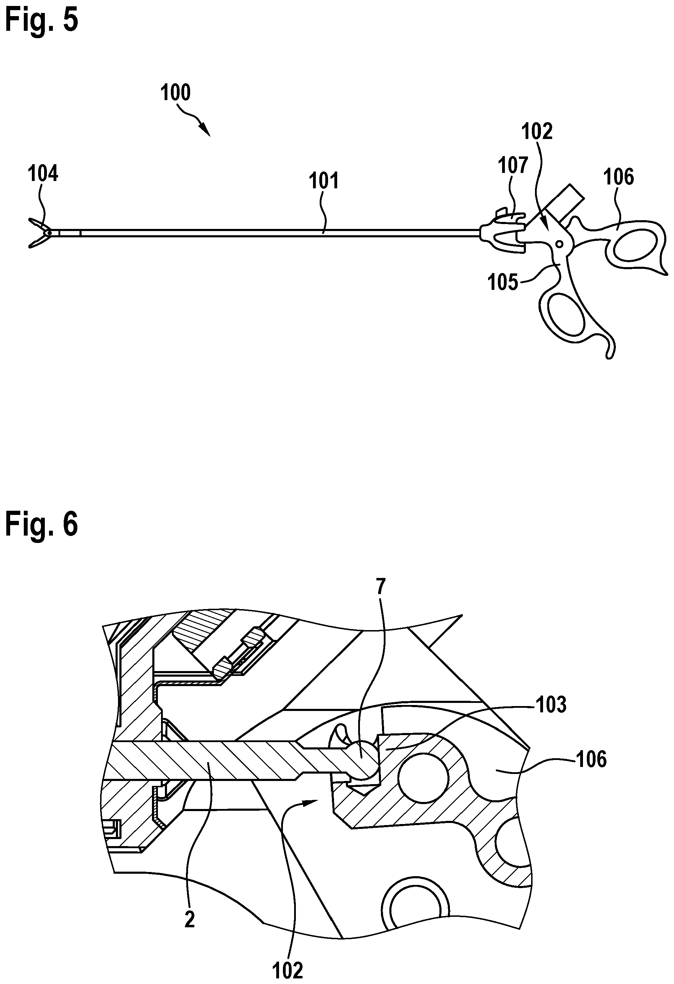

Fig. 5 zeigt eine Seitenansicht einer Ausführungsform eines handgeführten chirurgischen Instruments 100. Das chirurgische Instrument 100 umfasst ein Kraftübertragungselement (hier nicht dargestellt, siehe Figs. 1 bis 4), einen Rohrschaft 101, eine Betätigungsschnittstelle 102, ein Lagerelement (hier nicht dargestellt, siehe Fig. 6), ein Werkzeug bzw. Zubehör 104, einen Handgriff 105, einen beweglichen Griffschenkel 106 und eine Zubehörschnittstelle 107.

Das Kraftübertragungselement ist in dem Rohrschaft 101 aufgenommen und gelagert. Das Lagerelement sichert das Kraftübertragungselement gegen axiale Verschiebung gegenüber dem Rohrschaft 101 (vgl. Fig. 6). Das Werkzeug 104 ist hier als Greifer ausgebildet, welcher über das Kraftübertragungselement mit beiden Polen des elektrischen Stroms versorgt werden kann. Das chirurgische Instrument 100 ist hier zur händischen Führung durch einen Nutzer (z. B. Chirurg) mit einem Handgriff 105 ausgestattet. Alternativ kann das chirurgische Instrument 100 zur Führung durch einen Roboter mit einer entsprechenden Anschlussschnittstelle für den Roboter ausgestattet sein (nicht dargestellt). Der bewegliche Griffschenkel 106 dient der manuellen Krafteinleitung. Dabei wird die auf den Griffschenkel 106 aufgebrachte Kraft durch den Griffschenkel 106 auf das Kraftübertragungselement übertragen. Das Kraftübertragungselement überträgt wiederum die axiale Kraft auf das Werkzeug / Zubehör. Zusätzlich werden über das Kraftübertragungselement zwei elektrische Pole eines bipolaren Generators (nicht dargestellt) mit dem Werkzeug 104 verbunden. Über die Zubehörschnittstelle 107 kann das Werkzeug / Zubehör 104 mit dem Handgriff 105 lösbar mechanisch verbunden werden.

Fig. 6 zeigt einen Längsschnitt durch das handgeführte chirurgische Instrument 100 im Bereich eines Lagerelements 103 einer Betätigungsschnittstelle 102.

Das Lagerelement 103 greift ein kugelförmiges Formschlusselement 7 eines Kraftübertragungselements. Zudem ist ein Stab 2 des Kraftübertragungselements dargestellt, der mit seinem proximalen Ende an die Betätigungsschnittstelle 102 zur mechanischen Betätigung gekoppelt ist. Durch eine hebelartige Manipulation des beweglichen Griffschenkels 106 wird der Stab 2 axial relativ zu dem chirurgischen Instrument verschoben. Dabei ist das Lagerelement 103 zum Beispiel gabelartig ausgebildet, sodass zwei Schenkel des gabelartigen Lagerelements 103 das kugelförmige Formschlusselement formschlüssig umgreifen und den Stab 2 insbesondere ziehen können. Darüber hinaus kann das Lagerelement 103 das kugelförmige Formschlusselement axial drücken.

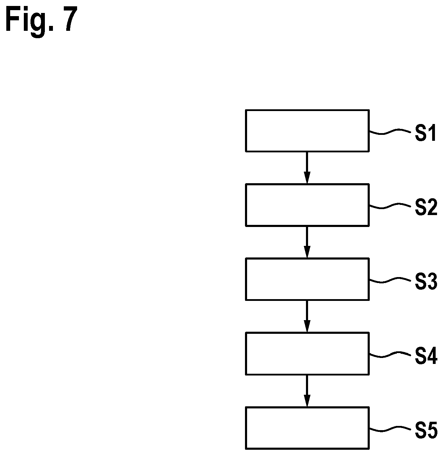

In Fig. 7 ist eine Ausführungsform des Verfahrens zur Herstellung des Kraftübertragungselements für chirurgische Instrumente, insbesondere eines Kraftübertragungselements 1 aus den Fig. 1 bis 4, schematisch dargestellt. Das Verfahren umfasst die Schritte Bereitstellen S1, Hindurchschieben S2, stoffschlüssiges Verbinden S3, stoffschlüssiges Verbinden S4 und Aufbringen S5.

Im Schritt des Bereitstellens S1 wird ein isolierender zur Kraftübertragung ausgebildeter Stab 2 bereitgestellt, der zumindest eine lokale Vertiefung 3 und ein Ende 6, insbesondere ein proximales Ende, mit einem elektrisch isolierten Formschlusselement 7 aufweist. Das Formschlusselement 7 ist zum Eingriff mit einem Lagerelement 103 eines chirurgischen Instruments 100 ausgebildet. Der Stab 2 weist einen Mittelabschnitt 8 und einen zwischen dem Mittelabschnitt 8 und dem Ende 6 angeordneten Kontaktabschnitt 9 auf, in welchem die zumindest eine Vertiefung 3 angeordnet ist.

Beim Schritt des Hindurchschiebens S2 wird eine Drahteinrichtung 11 durch eine in dem Kontaktabschnitt 9 vorgesehene Hülse 10 aus keramischem Werkstoff hindurchgeschoben. Die Drahteinrichtung 11 enthält zum Beispiel mehrere Zugdrähte. Die Zugdrähte 11 werden durch die Hülse 10 positioniert. Die Zugdrähte werden auf Anschlag in das proximale Ende bzw. Endstück des Stabs eingeführt. Darüber hinaus können etwa die hinteren 2/3 der Zugdrähte mit einem Klebstoff benetzt werden, bevor die Hülse aufgeschoben wird.

Der Schritt S3 umfasst das stoffschlüssige Verbinden der Zugdrähte 11 mit dem Formschlusselement 7. Die Zugdrähte 11 werden stirnseitig sowie durch eine Aussparung 13 des proximalen Endes, die sich in radialer Richtung erstreckt, verschweißt. Eine Stirnseite des proximalen Endes kann dabei vollflächig mit einem Klebstoff benetzt und mit der Hülse gepresst sein. Im Anschluss an das Schweißen können die stoffschlüssigen Verbindungen zum Beispiel bei etwa 100°C für ca. 15 min aushärten.

Nachdem der Kontaktabschnitt mit dem proximalen Ende stoffschlüssig verbunden S3 ist, werden die Zugdrähte 11 mit der aufgeschobenen Hülse 10 an den Mittelabschnitt 8 stoffschlüssig verbunden im Schritt S4, sodass der elektrische Leiter 5 innerhalb der Hülse 10 aufgenommen wird. Dabei wird der elektrische Leiter durch die Hülse gefädelt. Der elektrische Leiter kann etwa 6-10 cm länger vorgesehen sein als dies im montierten Zustand nötig ist. Folglich wird der elektrische Leiter erst im montierten Zustand auf eine geeignete Länge zugeschnitten. Die Hülse wird beispielsweise in den Mittelabschnitt eingeklebt. Die Zugdrähte werden mit einem Zusatzmittel an den Mittelabschnitt angeschweißt. Vorzugsweise wird dabei eine Temperaturentwicklung an der Schweißstelle beobachtet, um aufgrund der sehr engen Kabelführung eine Beschädigung des elektrischen Leiters durch den Schweißprozess zu vermeiden. Zum Beispiel wird nur entlang des Zugdrahtes geschweißt. Die Schweißstelle kann zusätzlich mit einem temperaturbeständigen Klebstoff und/oder einem Füllstoff versehen werden. Im Anschluss an das Schweißen und Kleben können die stoffschlüssigen Verbindungen zum Beispiel bei etwa 100°C für ca. 15 min aushärten.

Im Schritt des Aufbringens S5 wird eine Elektrode 4 aufgebracht, die sich über eine äußere Oberfläche der Vertiefung 3 und zumindest abschnittsweise in axialer Richtung entlang der Vertiefung 3 erstreckt. Die Elektrode 4 wird in Kontakt mit dem elektrischen Leiter 5 gebracht, der sich innerhalb des Stabs 2 erstreckt. Dabei kann das Aufbringen S5 der Elektrode 4 ein Abisolieren und Anschweißen, Löten und/oder Krimpen des elektrischen Leiters 5 an die Elektrode umfassen. Die Elektrode kann beispielsweise aus zwei Elektrodenhalbschalen bestehen. Insbesondere wird der elektrische Leiter an eine Innenseite der Elektrodenhalbschale angeschweißt.

Optional kann das Verfahren einen Schritt des Prüfens der elektrischen Verbindung/Isolation umfassen. Dabei wird ein Durchgangswiderstand von der Elektrodenhalbschale bis zu einer Elektrode im Werkzeug eines chirurgischen Instruments gemessen. Zudem wird ein Widerstand der Elektrodenhalbschale zum jeweiligen anderen Werkzeug sowie ein Widerstand der Elektroden gegenüber dem Kraftübertragungselement bzw. der Zugstange gemessen.

Nach dem Aufbringen S5 der Elektrodenhalbschalen kann eine Schweißnaht, optional mit Zusatzmaterial, erzeugt werden, um die Elektrodenhalbschalen zu verbinden. Alle Schweißstellen werden überschliffen. Hierbei ist zu beachten, dass die Elektrodenhalbschale nur durch die Schweißstelle an dem elektrischen Leiter gegenüber Rotation gesichert ist.

Ferner kann optional ein kapillar wirkender Klebstoff aufgetragen werden. Zum Beispiel wird das proximale Ende in den kapillar wirkenden Klebstoff getaucht, sodass durch die Kapillarwirkung Spalte zwischen den Elektrodenhalbschalen und der Hülse oder einem Kabelkanal verfüllt werden.

In der vorangegangenen detaillierten Beschreibung sind verschiedene Merkmale zur Verbesserung der Stringenz der Darstellung in einem oder mehreren Beispielen zusammengefasst worden. Es sollte dabei jedoch klar sein, dass die obige Beschreibung lediglich illustrativer, keinesfalls jedoch beschränkender Natur ist. Sie dient der Abdeckung aller Alternativen, Modifikationen und Äquivalente der verschiedenen Merkmale und Ausführungsbeispiele. Viele andere Beispiele werden dem Fachmann aufgrund seiner fachlichen Kenntnisse in Anbetracht der obigen Beschreibung sofort und unmittelbar klar sein.

Die Ausführungsbeispiele wurden ausgewählt und beschrieben, um die der Erfindung zugrundeliegenden Prinzipien und ihre Anwendungsmöglichkeiten in der Praxis bestmöglich darstellen zu können. Dadurch können Fachleute die Erfindung und ihre verschiedenen Ausführungsbeispiele in Bezug auf den beabsichtigten Einsatzzweck optimal modifizieren und nutzen. In den Ansprüchen sowie der Beschreibung werden die Begriffe "beinhaltend" und "aufweisend" als neutralsprachliche Begrifflichkeiten für die entsprechenden Begriffe "umfassend" verwendet. Weiterhin soll eine Verwendung der Begriffe "ein", "einer" und "eine" eine Mehrzahl derartig beschriebener Merkmale und Komponenten nicht grundsätzlich ausschließen.

### BEZUGSZEICHENLISTE

- 1: Kraftübertragungselement
- 2: Stab
- 3: Vertiefung
- 4: Elektrode
- 5: elektrischer Leiter
- 6: Ende
- 7: Formschlusselement
- 8: Mittelabschnitt
- 9: Kontaktabschnitt
- 10: Hülse
- 11: Drahteinrichtung
- 12: Bohrung
- 13: Aussparung

- 100: chirurgisches Instrument
- 101: Rohrschaft
- 102: Betätigungsschnittstelle
- 103: Lagerelement
- 104: Werkzeug / Zubehör
- 105: Handgriff
- 106: beweglicher Griffschenkel
- 107: Zubehörschnittstelle

- S1: Bereitstellen
- S2: Hindurchschieben
- S3: stoffschlüssiges Verbinden
- S4: stoffschlüssiges Verbinden
- S5: Aufbringen

## Patentansprüche

1. Kraftübertragungselement (1) für chirurgische Instrumente, insbesondere Zugstange für ein endoskopisches Instrument, mit:
einem potentialfreien, zur Kraftübertragung ausgebildeten Stab (2), der zumindest eine lokale Vertiefung (3) aufweist; und
einer Elektrode (4), die sich über eine äußere Oberfläche der Vertiefung (3) und zumindest abschnittsweise in axialer Richtung entlang der Vertiefung (3) erstreckt, wobei die Elektrode (4) in Kontakt mit einem elektrischen Leiter (5) ist, der sich innerhalb des Stabs (2) erstreckt;
wobei ein Ende (6), insbesondere ein proximales Ende, des Stabs (2) ein elektrisch isoliertes Formschlusselement (7) aufweist, das zum Eingriff mit einem Lagerelement (103) eines chirurgischen Instruments (100) ausgebildet ist.

2. Kraftübertragungselement (1) nach Anspruch 1, **dadurch gekennzeichnet, dass** der Stab (2) einen Mittelabschnitt (8) und einen zwischen dem Mittelabschnitt (8) und dem Ende (6) angeordneten Kontaktabschnitt (9) aufweist, in welchem die zumindest eine Vertiefung (3) angeordnet ist, wobei der Kontaktabschnitt (9) eine Hülse (10) aus keramischem Werkstoff aufweist.

3. Kraftübertragungselement (1) nach Anspruch 2, **dadurch gekenn** - **zeichnet,** dass der Kontaktabschnitt (9) eine Drahteinrichtung (11) zur Kraftübertragung zwischen dem Mittelabschnitt (8) und dem Ende (6) aufweist, wobei sich die Drahteinrichtung (11) in axialer Richtung entlang des Stabs (2) erstreckt und zumindest abschnittsweise von der Hülse (10) umgeben ist.

4. Kraftübertragungselement (1) nach Anspruch 3, **dadurch gekenn** - **zeichnet,** dass die Drahteinrichtung (11) mit dem Formschlusselement (7) und dem Mittelabschnitt (8) des Stabs (2) jeweils stoffschlüssig verbunden ist, wobei die Hülse (10) durch die Drahteinrichtung (11) zwischen dem Formschlusselement (7) und dem Mittelabschnitt (8) zumindest formschlüssig befestigt ist.

5. Kraftübertragungselement (1) nach Anspruch 4, **dadurch gekenn** - **zeichnet,** dass die Drahteinrichtung (11) mit dem Formschlusselement (7) und dem Mittelabschnitt (8) jeweils stoffschlüssig, insbesondere mittels einer Schweißverbindung oder mittels eines kapillar wirkenden Klebstoffs, verbunden ist.

6. Kraftübertragungselement (1) nach einem der Ansprüche 2 bis 5, **dadurch gekennzeichnet, dass** die Hülse (10) durch ein generatives Fertigungsverfahren oder durch Spritzgießen einteilig hergestellt ist.

7. Kraftübertragungselement (1) nach einem der vorstehenden Ansprüche, **dadurch gekennzeichnet, dass** die Vertiefung (3) wenigstens einen im Wesentlichen ebenen Abschnitt parallel zu der axialen Richtung des Stabs (2) aufweist, welcher gegenüber einer äußeren Oberfläche des Stabs in radialer Richtung weiter innen liegt und mit der Elektrode (4) aneinander anliegend, insbesondere flächig anliegend, kontaktiert ist.

8. Kraftübertragungselement (1) nach einem der vorstehenden Ansprüche, **dadurch gekennzeichnet, dass** das Formschlusselement (7) kugelförmig ausgebildet ist.

9. Chirurgisches Instrument (100), mit:
einem Kraftübertragungselement (1) gemäß einem der vorstehenden Ansprüche;
einem Rohrschaft (101), in welchem das Kraftübertragungselement (1) aufgenommen ist;
einer Betätigungsschnittstelle (102), die zum Betätigen des Kraftübertragungselements (1) ausgebildet ist;
einem Lagerelement (103), das in der Betätigungsschnittstelle (102) integriert ist und das Formschlusselement (7) des Kraftübertragungselements (1) koppelt; und
einem Werkzeug (104), das mittels des Kraftübertragungselements (1) bewegbar ist.

10. Chirurgisches Instrument (10) nach Anspruch 9, **dadurch gekenn** - **zeichnet,** dass der Rohrschaft (101) als Rundrohr ausgebildet ist, wobei der Stab (2) des Kraftübertragungselements (1) als Rundstab ausgebildet ist und sich die Hülse (10) zumindest abschnittsweise zirkulär entlang der Oberfläche des Stabs (2) erstreckt, und wobei das Kraftübertragungselement (1) konzentrisch in dem Rohrschaft (101) angeordnet ist.

11. Verfahren zur Herstellung eines Kraftübertragungselements für chirurgische Instrumente, insbesondere eines Kraftübertragungselements (1) gemäß einem der Ansprüche 1 bis 8, umfassend die folgenden Schritte:
Bereitstellen (S1) eines potentialfreien, zur Kraftübertragung ausgebildeten Stabs (2), der zumindest eine lokale Vertiefung (3) und ein Ende (6), insbesondere ein proximales Ende, mit einem elektrisch isolierten Formschlusselement (7) aufweist, das zum Eingriff mit einem Lagerelement (103) eines chirurgischen Instruments (100) ausgebildet ist; und
Aufbringen (S5) einer Elektrode (4), die sich über eine äußere Oberfläche der Vertiefung (3) und zumindest abschnittsweise in axialer Richtung entlang der Vertiefung (3) erstreckt, wobei die Elektrode (4) in Kontakt mit einem elektrischen Leiter (5) gebracht wird, der sich innerhalb des Stabs (2) erstreckt.

12. Verfahren nach Anspruch 11, **dadurch gekennzeichnet, dass** das Aufbringen (S5) der Elektrode (4) ein Abisolieren und Anschweißen, Löten und/oder Krimpen des elektrischen Leiters (5) an die Elektrode umfasst.

13. Verfahren nach Anspruch 11 oder 12, **dadurch gekennzeichnet, dass** der Stab (2) einen Mittelabschnitt (8) und einen zwischen dem Mittelabschnitt (8) und dem Ende (6) angeordneten Kontaktabschnitt (9) aufweist, in welchem die zumindest eine Vertiefung (3) angeordnet ist, wobei eine Drahteinrichtung (11) durch eine in dem Kontaktabschnitt (9) vorgesehene Hülse (10) aus keramischem Werkstoff hindurchgeschoben (S2) wird.

14. Verfahren nach Anspruch 13, **dadurch gekennzeichnet, dass** das Verfahren ferner den Schritt stoffschlüssiges Verbinden (S3) der Drahteinrichtung (11) mit dem Formschlusselement (7) umfasst.

15. Verfahren nach Anspruch 14, **dadurch gekennzeichnet, dass** nach dem stoffschlüssigen Verbinden (S3) die Drahteinrichtung (11) mit der aufgeschobenen Hülse (10) an den Mittelabschnitt (8) stoffschlüssig verbunden (S4) wird, sodass der elektrische Leiter (5) innerhalb der Hülse (10) aufgenommen wird.

## Claims

1. A force transmission element (1) for surgical instruments, in particular a pull rod for an endoscopic instrument, comprising:
a potential-free rod (2) which is designed for force transmission and which has at least one localised depression (3); and
an electrode (4), which extends over an outer surface of the depression (3) and at least in sections in the axial direction along the depression (3), wherein the electrode (4) is in contact with an electrical conductor (5), which extends inside the rod (2);
wherein one end (6), in particular a proximal end, of the rod (2) has an electrically insulated positive-locking element (7) which is designed to engage with a bearing element (103) of a surgical instrument (100).

2. The force transmission element (1) according to claim 1, **characterised in that** the rod (2) has a central section (8) and a contact section (9) which is arranged between the central section (8) and the end (6) and in which the at least one depression (3) is arranged, wherein the contact section (9) has a sleeve (10) made of ceramic material.

3. The force transmission element (1) according to claim 2, **characterised in that** the contact section (9) has a wire device (11) for force transmission between the central section (8) and the end (6), wherein the wire device (11) extends in the axial direction along the rod (2) and is surrounded at least in sections by the sleeve (10).

4. The force transmission element (1) according to claim 3, **characterised in that** the wire device (11) is connected in each case to the positive-locking element (7) and to the central section (8) of the rod (2) in a materially-bonded manner, wherein the sleeve (10) is fastened at least in a positive-locking manner by the wire device (11) between the positive-locking element (7) and the central section (8).

5. The force transmission element (1) according to claim 4, **characterised in that** the wire device (11) is connected to the positive-locking element (7) and to the central section (8) in each case in a materially-bonded manner, in particular by means of a welded connection or by means of a capillary-acting adhesive.

6. The force transmission element (1) according to one of claims 2 to 5, **characterised in that** the sleeve (10) is produced in one piece by a generative manufacturing process or by injection moulding.

7. The force transmission element (1) according to one of the preceding claims, **characterised in that** the depression (3) has at least one substantially planar section parallel to the axial direction of the rod (2), which lies further inward in the radial direction relative to an outer surface of the rod and is in abutting contact with the electrode (4), in particular in surface-to-surface contact.

8. The force transmission element (1) according to one of the preceding claims, **characterised in that** the positive-locking element (7) is spherical in shape.

9. A surgical instrument (100), comprising:
a force transmission element (1) according to one of the preceding claims;
a tubular shaft (101) in which the force transmission element (1) is received;
an actuating interface (102) which is designed to actuate the force transmission element (1);
a bearing element (103), which is integrated in the actuating interface (102) and which couples the positive-locking element (7) of the force transmission element (1); and
a tool (104) which is movable by means of the force transmission element (1).

10. The surgical instrument (10) according to claim 9, **characterised in that** the tubular shaft (101) is in the form of a round tube, wherein the rod (2) of the force transmission element (1) is in the form of a round rod and the sleeve (10) extends at least in sections circularly along the surface of the rod (2), and wherein the force transmission element (1) is arranged concentrically in the tubular shaft (101).

11. A method for manufacturing a force transmission element for surgical instruments, in particular a force transmission element (1) according to one of claims 1 to 8, comprising the following steps:
Providing (S1) a potential-free rod (2) which is designed for force transmission and which has at least one localised depression (3) and one end (6), in particular a proximal end, with an electrically insulated positive-locking element (7) which is designed to engage with a bearing element (103) of a surgical instrument (100); and
Applying (S5) an electrode (4), which extends over an outer surface of the depression (3) and at least in sections in the axial direction along the depression (3), wherein the electrode (4) is brought into contact with an electrical conductor (5), which extends inside the rod (2).

12. The method according to claim 11, **characterised in that** applying (S5) the electrode (4) comprises stripping and welding, soldering and/or crimping the electrical conductor (5) to the electrode.

13. The method according to claim 11 or 12, **characterised in that** the rod (2) has a central section (8) and a contact section (9) which is arranged between the central section (8) and the end (6) and in which the at least one depression (3) is arranged, wherein a wire device (11) is pushed (S2) through a sleeve (10), made of ceramic material, provided in the contact section (9).

14. The method according to claim 13, **characterised in that** the method further comprises the step of materially bonding (S3) the wire device (11) to the positive-locking element (7).

15. The method according to claim 14, **characterised in that**, after materially bonding (S3), the wire device (11) is materially bonded (S4) to the central section (8) with the sleeve (10) pushed onto it, such that the electrical conductor (5) is received inside the sleeve (10).

## Revendications

1. Élément de transmission de force (1) pour instruments chirurgicaux, en particulier tige de traction pour un instrument endoscopique, comportant :
une barre (2) sans potentiel, conçue pour la transmission de force, qui présente au moins un renfoncement (3) local ; et
une électrode (4) qui s'étend sur une surface extérieure du renfoncement (3) et, au moins par sections, dans une direction axiale le long du renfoncement (3), dans lequel l'électrode (4) est en contact avec un conducteur électrique (5) qui s'étend à l'intérieur de la barre (2) ;
dans lequel une extrémité (6), en particulier une extrémité proximale, de la barre (2) présente un élément de complémentarité de forme (7) isolé électriquement, qui est conçu pour venir en prise avec un élément de palier (103) d'un instrument chirurgical (100).

2. Élément de transmission de force (1) selon la revendication 1, **caractérisé en ce que** la barre (2) présente une section centrale (8) et une section de contact (9) agencée entre la section centrale (8) et l'extrémité (6), dans laquelle est agencé l'au moins un renfoncement (3), dans lequel la partie de contact (9) présente un manchon (10) en matériau céramique.

3. Élément de transmission de force (1) selon la revendication 2, **caractérisé en ce que** la section de contact (9) présente un équipement à fil (11) pour la transmission de force entre la section centrale (8) et l'extrémité (6), dans lequel l'équipement à fil (11) s'étend dans une direction axiale le long de la barre (2) et est entouré au moins par sections par le manchon (10).

4. Élément de transmission de force (1) selon la revendication 3, **caractérisé en ce que** l'équipement à fil (11) est relié par une complémentarité de matière respectivement à l'élément de complémentarité de forme (7) et à la section centrale (8) de la barre (2), dans lequel le manchon (10) est fixé par l'équipement à fil (11) entre l'élément de complémentarité de forme (7) et la section centrale (8) au moins par une complémentarité de forme.

5. Élément de transmission de force (1) selon la revendication 4, **caractérisé en ce que** l'équipement à fil (11) est relié à l'élément de complémentarité de forme (7) et à la section centrale (8) respectivement par complémentarité de matière, en particulier par le biais d'une soudure ou par le biais d'une colle à action capillaire.

6. Élément de transmission de force (1) selon l'une des revendications 2 à 5, **caractérisé en ce que** le manchon (10) est fabriqué en une seule pièce par un procédé de fabrication génératif ou par moulage par injection.

7. Élément de transmission de force (1) selon l'une des revendications précédentes, **caractérisé en ce que** le renfoncement (3) présente au moins une section sensiblement plane parallèle à la direction axiale de la barre (2), laquelle est située plus à l'intérieur dans la direction radiale par rapport à une surface extérieure de la barre et est en contact avec l'électrode (4) de manière adjacente, en particulier de manière adjacente sur toute sa surface.

8. Élément de transmission de force (1) selon l'une des revendications précédentes, **caractérisé en ce que** l'élément de complémentarité de forme (7) est conçu de forme sphérique.

9. Instrument chirurgical (100), comportant :
un élément de transmission de force (1) selon l'une des revendications précédentes ;
un arbre tubulaire (101) dans lequel l'élément de transmission de force (1) est logé ;
une interface d'actionnement (102), qui est conçue pour actionner l'élément de transmission de force (1) ;
un élément de palier (103), qui est intégré dans l'interface d'actionnement (102) et qui accouple l'élément de complémentarité de forme (7) de l'élément de transmission de force (1) ; et
un outil (104), qui peut être déplacé par le biais de l'élément de transmission de force (1).

10. Instrument chirurgical (10) selon la revendication 9, **caractérisé en ce que** l'arbre tubulaire (101) est conçu comme un tube rond, dans lequel la barre (2) de l'élément de transmission de force (1) est conçue comme une barre ronde et le manchon (10) s'étend au moins par sections de manière circulaire le long de la surface de la barre (2), et dans lequel l'élément de transmission de force (1) est agencé de manière concentrique dans l'arbre tubulaire (101).

11. Procédé de fabrication d'un élément de transmission de force pour des instruments chirurgicaux, en particulier d'un élément de transmission de force (1) selon l'une des revendications 1 à 8, comprenant les étapes suivantes :
mise à disposition (S1) d'une barre (2) sans potentiel, conçue pour la transmission de force, qui présente au moins un renfoncement (3) local et une extrémité (6), en particulier une extrémité proximale, comportant un élément de complémentarité de forme (7) électriquement isolé, qui est conçu pour venir en prise avec un élément de palier (103) d'un instrument chirurgical (100) ; et
Application (S5) d'une électrode (4) qui s'étend sur une surface extérieure du renfoncement (3) et au moins par sections dans une direction axiale le long du renfoncement (3), dans laquelle l'électrode (4) est mise en contact avec un conducteur électrique (5) qui s'étend à l'intérieur de la barre (2).

12. Procédé selon la revendication 11, **caractérisé en ce que** l'application (S5) de l'électrode (4) comprend le dénudage et le soudage, le brasage et/ou le sertissage du conducteur électrique (5) sur l'électrode.

13. Procédé selon la revendication 11 ou 12, **caractérisé en ce que** la barre (2) présente une section centrale (8) et une section de contact (9) agencée entre la section centrale (8) et l'extrémité (6), dans laquelle est agencé l'au moins un renfoncement (3), dans lequel un équipement à fil (11) est poussé (S2) à travers un manchon (10) en matériau céramique prévu dans la section de contact (9).

14. Procédé selon la revendication 13, **caractérisé en ce que** le procédé comprend en outre l'étape de liaison par complémentarité de matière (S3) de l'équipement à fil (11) avec l'élément de complémentarité de forme (7).

15. Procédé selon la revendication 14, **caractérisé en ce qu'**après la liaison par complémentarité de matière (S3), l'équipement à fil (11) est relié par complémentarité de matière (S4) à la section centrale (8) avec le manchon (10) enfoncé, de sorte que le conducteur électrique (5) est logé à l'intérieur du manchon (10).
